# EUROPEAN PATENT APPLICATION

(11) **EP 4 332 213 A1**
(43) Date of publication of application: **06.03.2024**
(21) Application number: 22795395.7
(22) Date of filing: 24.03.2022
(51) Int. Cl.: C12N 1/20

(54) **GROWTH PROMOTER FOR LACTIC ACID COCCUS**

(30) Priority: 28.04.2021 JP 2021075882; 18.06.2021 JP 2021101500
(71) Applicant: KIRIN HOLDINGS KABUSHIKI KAISHA, Nakano-ku, Tokyo 164-0001 (JP)
(72) Inventor: HIROSE, Yu, Tokyo 164-0001 (JP); MURAKAWA, Hazuki, Tokyo 164-0001 (JP)
(74) Representative: Hoffmann Eitle
(86) International application number: PCT/JP2022/013938
(87) International publication number: WO 2022/230476

(57) **Abstract**

An object of the present invention is to provide a growth promoter for a lactic acid coccus. According to the present invention, there is provided a growth promoter for a lactic acid coccus, comprising a dicarboxylic acid or a salt thereof as an active ingredient. The dicarboxylic acid is preferably an aliphatic dicarboxylic acid having 4 to 6 carbon atoms. The lactic acid coccus is preferably a bacterium belonging to the genus *Lactococcus.* According to the present invention, the growth of a lactic acid coccus can be promoted by including a dicarboxylic acid in a medium, and, therefore, the present invention is advantageous in that the lactic acid coccus can be cultivated more easily.

## Description

### CROSS-REFERENCE TO RELATED APPLICATION

The present application enjoys the benefit of priority from the prior Japanese Patent Application No. 2021-75882 filed on April 28, 2021 and the prior Japanese Patent Application No. 2021-101500 filed on June 18, 2021, the entire disclosures of which are incorporated herein by reference.

### TECHNICAL FIELD

The present invention relates to a growth promoter for a lactic acid coccus. The present invention also relates to a medium for a lactic acid coccus, a method for cultivation of a lactic acid coccus, and a method for promoting the growth of a lactic acid coccus.

### BACKGROUND ART

LB medium and MRS medium are generally used in the cultivation of lactic acid cocci. However, sufficient growth of lactic acid cocci may not be achieved, depending on the type of medium. The methods described in Patent Documents 1 and 2 have been known so far as methods for efficient cultivation of a lactic acid coccus.

### Reference List

### Patent Documents

Patent Document 1: JP 2020-137456 A
Patent Document 2: JP 2020-22393 A

### SUMMARY OF THE INVENTION

An object of the present invention is to provide a novel growth promoter for a lactic acid coccus. Another object of the present invention is to provide a novel medium for a lactic acid coccus, a novel method for cultivation of a lactic acid coccus, and a novel method for promoting the growth of a lactic acid coccus.

The present inventors have found that, in the cultivation of a lactic acid coccus, the growth of the lactic acid coccus is promoted by including a dicarboxylic acid or a salt thereof in a medium. The present invention is based on this finding.

According to the present invention, the following inventions are provided.
[1] A growth promoter for a lactic acid coccus, comprising a dicarboxylic acid and/or a salt thereof as an active ingredient.
[2] The growth promoter according to [1], wherein the dicarboxylic acid is an aliphatic dicarboxylic acid having 4 to 6 carbon atoms.
[3] The growth promoter according to [1] or [2], wherein the dicarboxylic acid and/or salt thereof is one or more selected from the group consisting of malic acid, α-ketoglutaric acid and salts thereof.
[4] The growth promoter according to any one of [1] to [3], wherein the lactic acid coccus is a bacterium belonging to the genus *Lactococcus.*
[5] The growth promoter according to any one of [1] to [4], wherein the lactic acid coccus is *Lactococcus lactis* subsp. *lactis* JCM5805.
[6] The growth promoter according to any one of [1] to [5], wherein the dicarboxylic acid and/or salt thereof is used at a concentration of 0.01 to 8% by mass in a medium.
[7] The growth promoter according to any one of [1] to [6], which is added to a medium that is substantially free of meat extract.
[8] A medium for a lactic acid coccus, comprising a dicarboxylic acid and/or a salt thereof.
[9] The medium according to [8], which contains the dicarboxylic acid and/or salt thereof at a concentration of 0.01 to 8% by mass.
[10] The medium according to [8] or [9], which is substantially free of meat extract.
[11] A method for cultivation of a lactic acid coccus, comprising the step of cultivation of a lactic acid coccus in the medium according to any one of [8] to [10].
[12] A method for promoting the growth of a lactic acid coccus, comprising the step of adding a dicarboxylic acid and/or a salt thereof to a medium for a lactic acid coccus.
[13] Use of a dicarboxylic acid and/or a salt thereof as a growth promoter for a lactic acid coccus.

According to the present invention, the growth of a lactic acid coccus can be promoted by including a dicarboxylic acid or a salt thereof in a medium to cultivate the lactic acid coccus, and, therefore, the present invention is advantageous in that the lactic acid coccus can be cultivated easily and efficiently. The present invention is advantageous also in that the growth of a lactic acid coccus can be promoted by including a dicarboxylic acid or a salt thereof also in a medium that is free of meat extract.

### BRIEF DESCRIPTION OF THE DRAWINGS

FIG. 1 shows the amount of a *Lactococcus lactis* subsp. *lactis* at each concentration when α-ketoglutaric acid or a salt thereof or malic acid is added to MRS medium under anaerobic cultivation conditions (FIG. 1A: α-ketoglutaric acid (αKG), FIG. 1B: α-ketoglutaric acid disodium salt (αKG2Na), and FIG. 1C: malic acid). The control represents the case where no dicarboxylic acid is added.
FIG. 2 shows the amount of a *Lactococcus lactis* subsp. *lactis* at each concentration when α-ketoglutaric acid or malic acid is added to MRS medium under aerobic cultivation conditions (FIG. 2A: α-ketoglutaric acid (αKG) and FIG. 2B: malic acid). The control represents the case where no dicarboxylic acid is added.
FIG. 3 shows the amount of a lactic acid coccus at each concentration when α-ketoglutaric acid (αKG) or malic acid is added to GM17 medium under anaerobic cultivation conditions. The control represents the case where no dicarboxylic acid is added.
FIG. 4 shows the amount of a *Lactococcus lactis* subsp. *lactis* at each concentration when α-ketoglutaric acid or malic acid is added to GM17 medium under aerobic cultivation conditions (FIG. 4A: α-ketoglutaric acid (αKG) and FIG. 4B: malic acid). The control represents the case where no dicarboxylic acid is added.
FIG. 5 shows the amounts of various lactic acid cocci at each concentration when α-ketoglutaric acid (αKG) is added to MRS medium under anaerobic cultivation conditions. The control represents the case where no dicarboxylic acid is added.
FIG. 6 shows the amount of a lactic acid-producing bacillus belonging to the genus *Lactobacillus* at each concentration when α-ketoglutaric acid (αKG) is added to MRS medium under anaerobic cultivation conditions. The control represents the case where no dicarboxylic acid is added.
FIG. 7 shows the amount of a *Lactococcus lactis* subsp. *lactis* at each concentration when citric acid is added to MRS medium under anaerobic cultivation conditions. The control represents the case where no citric acid is added.
FIG. 8 shows the amounts of lactic acid cocci belonging to the genus *Pediococcus* at each concentration when α-ketoglutaric acid (αKG) or malic acid is added to MRS medium under anaerobic cultivation conditions. The control represents the case where no dicarboxylic acid is added.
FIG. 9 shows the amounts of various lactic acid cocci at each concentration when α-ketoglutaric acid (αKG) is added to MRS medium free of meat extract under anaerobic cultivation conditions. The control represents the case where no dicarboxylic acid is added.
FIG. 10 shows the amounts of various lactic acid cocci in a medium free of meat extract (without meat extract) and a medium containing meat extract (with meat extract) under anaerobic cultivation conditions.

### DETAILED DESCRIPTION OF THE INVENTION

The growth promoter of the present invention can be used in the cultivation of a lactic acid coccus. The lactic acid coccus as the target for growth promotion is not particularly limited, and examples thereof include lactic acid cocci belonging to the genus *Lactococcus,* the genus *Leuconostoc,* the genus *Pediococcus,* and the genus *Streptococcus.*

Examples of lactic acid cocci belonging to the genus *Lactococcus* include *Lactococcus lactis* subsp. *lactis, Lactococcus garvieae, Lactococcus lactis* subsp. *cremoris,* and *Lactococcus lactis* subsp. *hordniae,* and *Lactococcus lactis* subsp. *lactis* is preferable.

Specific examples of bacteria belonging to the genus *Lactococcus* include *Lactococcus lactis* subsp. *lactis* JCM5805, *Lactococcus lactis* subsp. *lactis* NBRC12007, *Lactococcus lactis* subsp. *lactis* NRIC1150, *Lactococcus lactis* subsp. *lactis* JCM20101, *Lactococcus lactis* subsp. *lactis* JCM7638, *Lactococcus lactis* subsp. *lactis* ATCC11454, *Lactococcus garvieae* NBRC100934, *Lactococcus lactis* subsp. *cremoris* JCM16167, *Lactococcus lactis* subsp. *cremoris* NBRC100676, *Lactococcus lactis* subsp. *hordniae* JCM1180, and *Lactococcus lactis* subsp. *hordniae* JCM11040, and *Lactococcus lactis* subsp. *lactis* JCM5805 is preferable.

Examples of bacteria belonging to the genus *Leuconostoc* include *Leuconostoc lactis.* Specific examples of bacteria belonging to the genus *Leuconostoc* include *Leuconostoc lactis* NBRC12455.

Examples of bacteria belonging to the genus *Pediococcus* include *Pediococcus acidilactici, Pediococcus pentosaceus, Pediococcus cellicola, Pediococcus claussenii, Pediococcus damnosus, Pediococcus ethanolidurans, Pediococcus inopinatus, Pediococcus parvulus,* and *Pediococcus stilesii.* Specific examples of bacteria belonging to the genus *Pediococcus* include *Pediococcus acidilactici* JCM8797 and *Pediococcus damnosus* JCM5886.

Examples of bacteria belonging to the genus *Streptococcus* include *Streptococcus thermophilus.*

Among the above lactic acid coccus strains, the JCM bacterial strains are available from Microbe Division, RIKEN BioResource Research Center (3-1-1 Koyadai, Tsukuba, Ibaraki); the NBRC bacterial strains are available from Biological Resource Center, National Institute of Technology and Evaluation (2-5-8 Kazusa-Kamatari, Kisarazu, Chiba); the NRIC bacterial strains are available from NODAI Culture Collection Center, Tokyo University of Agriculture (1-1-1 Sakuragaoka, Setagaya, Tokyo); and the ATCC bacterial strains are available from American Type Culture Collection (U.S.A.).

The *Lactococcus lactis* subsp. *lactis* JCM5805 strain is available from Microbe Division, RIKEN BioResource Research Center as described above, but, in the present invention, the same bacterial strain as the JCM5805 strain, that is stored at a culture collection other than Microbe Division, RIKEN BioResource Research Center, can be used. Specifically, the same bacterial strain as the JCM5805 strain is available from Biological Resource Center, National Institute of Technology and Evaluation (2-5-8 Kazusa-Kamatari, Kisarazu, Chiba), NODAI Culture Collection Center, Tokyo University of Agriculture (1-1-1 Sakuragaoka, Setagaya, Tokyo), American Type Culture Collection (U.S.A.), and the like.

The dicarboxylic acid, which is the active ingredient of the growth promoter of the present invention, is not particularly limited as long as it is a compound having two carboxyl groups in its structure. An aliphatic dicarboxylic acid can be preferably used. The hydrocarbon chain that constitutes the aliphatic dicarboxylic acid is preferably a saturated hydrocarbon chain. The aliphatic dicarboxylic acid may additionally have an oxo group (=O) and/or a hydroxyl group (-OH) on the carbon atoms of its hydrocarbon chain. In the present invention, the aliphatic dicarboxylic acid is preferably an aliphatic dicarboxylic acid having 4 to 6 carbon atoms, more preferably an aliphatic dicarboxylic acid having 4 or 5 carbon atoms. The aliphatic dicarboxylic acid having 4 to 6 carbon atoms includes, for example, a ketoglutaric acid (α-ketoglutaric acid (sometimes referred to as "αKG" herein), β-ketoglutaric acid (acetone dicarboxylic acid)), malic acid, adipic acid, oxalic acid, succinic acid, maleic acid, and glutaric acid, and is preferably either or both of α-ketoglutaric acid and malic acid.

In the present invention, the dicarboxylic acid may be in the form of a salt, and examples of the salt include metal salts and ammonium salts. Examples of the metal salts include alkali metal salts such as sodium salts and potassium salts. Examples of the ammonium salts include salts of ammonium, tetramethylammonium and the like. Examples of the salt of dicarboxylic acid include α-ketoglutaric acid sodium salt and malic acid sodium salt. The growth promoter of the present invention can contain either or both of a dicarboxylic acid and its salt as the active ingredient.

The lower limit value (or more or more than) of the concentration (solid content concentration) of the dicarboxylic acid and/or salt thereof in a medium can be set to 0.01% by mass, 0.1% by mass, 0.25% by mass, or 0.5% by mass, and the upper limit value (or less or less than) thereof can be set to 8% by mass, 4% by mass, or 2% by mass. These lower and upper limit values can be combined arbitrarily. The range of the concentration of the dicarboxylic acid and/or salt thereof in the medium can be set to, for example, 0.01 to 8% by mass, 0.1 to 4% by mass, 0.25 to 4% by mass, 0.5 to 4% by mass, 0.5 to 3% by mass, or 0.5 to 2% by mass. The growth promoter of the present invention can promote the growth of a lactic acid coccus and efficiently cultivate the lactic acid coccus by setting the concentration of the dicarboxylic acid in the medium to fall within the concentration range defined above.

The growth promoter of the present invention can be added to a medium for cultivation of a lactic acid coccus for use. The medium that can be used in the present invention is not particularly limited as long as it is a medium used in the cultivation of a lactic acid coccus, and examples thereof include MRS medium (deMan, Rogosa & Sharpe medium), M17 medium, and LB medium. The growth promoter of the present invention may be added to the medium during cultivation of a lactic acid coccus, or may be added in advance before cultivation of a lactic acid coccus. The medium that can be used in the present invention can contain meat extract as a supplementary nutritional ingredient. Examples of the meat extract include beef extract, pork extract, and chicken extract. On the other hand, the medium that can be used in the present invention is preferably substantially free of meat extract, more preferably free of meat extract, from the viewpoint of environmental load, cost, food safety, and the like. Examples of the case where the medium is "substantially free of" meat extract include cases where the content of the meat extract is less than 0.8% by mass, less than 0.5% by mass, less than 0.3% by mass, less than 0.1% by mass, less than 0.05% by mass, or less than 0.01% by mass. The form of the meat extract to be used include, for example, extract liquid, powder, and granule. The content of the meat extract in the medium is calculated by dividing the solid content mass, which is a pure extract content, of the meat extract, by the mass of the medium and multiplying the obtained value by 100. The medium which is free of meat extract can be appropriately prepared without using meat extract in the raw material for the medium exemplified above. The growth promoter of the present invention can be added to the medium which is free of meat extract for use.

The conditions for cultivation of a lactic acid coccus using a medium added with the growth promoter of the present invention are not particularly limited, and normal conditions for cultivation of a lactic acid coccus can be employed.

The period for cultivation of a lactic acid coccus can be set to 20 to 48 hours, and is preferably 20 to 30 hours from the viewpoint of a growth phase.

The lower limit value (or higher or higher than) of the temperature for cultivation of a lactic acid coccus can be set to 20°C, 25°C, or 28°C, and the upper limit value (or lower or lower than) thereof can be set to 40°C, 35°C, or 30°C. These lower and upper limit values can be combined arbitrarily, and the cultivation temperature can be set to, for example, a range of 20 to 40°C, 20 to 35°C, 25 to 35°C, or 30 to 35°C, most preferably about 30°C.

The cultivation of a lactic acid coccus may be either liquid cultivation or solid cultivation. The liquid cultivation can be performed using a fermenter whose conditions can be controlled, and examples thereof include stirring cultivation, aeration cultivation, shaking cultivation, and stationary cultivation. The cultivation may be performed under either anaerobic conditions or aerobic conditions. The stirring speed and aeration rate are appropriately selected according to the type of microbial cells and the cultivation conditions.

The pH of the medium that is used in the present invention may be any pH that does not affect the growth of a lactic acid coccus, and can be set to, for example, a range of 6.0 to 6.7. The pH of the medium can be adjusted appropriately, and, for example, can be adjusted by adding a base such as sodium hydroxide or an acid such as phosphoric acid to the medium.

A culture of lactic acid coccus can be obtained by cultivating a lactic acid coccus in a medium added with the growth promoter of the present invention. The obtained lactic acid coccus culture can be blended in foods and drinks as it is or after treatments such as concentration, dilution, drying, isolation and purification. That is, the growth promoter of the present invention can be used to promote the growth of a lactic acid coccus for the purpose of application to foods and drinks, and, from this viewpoint, a dicarboxylic acid and/or a salt thereof acceptable as a food can be used as the active ingredient in the present invention.

According to another aspect of the present invention, there is provided a medium for a lactic acid coccus, containing a dicarboxylic acid and/or a salt thereof. The medium for a lactic acid coccus of the present invention can be implemented according to the descriptions regarding the growth promoter of the present invention. The medium for a lactic acid coccus of the present invention is advantageous in that it promotes the growth of a lactic acid coccus, and thus enables efficient cultivation of the lactic acid coccus.

According to another aspect of the present invention, there is provided a method for cultivation of a lactic acid coccus, comprising the step of cultivation of a lactic acid coccus in the medium of the present invention. The cultivation method of the present invention can be carried out according to the descriptions regarding the growth promoter of the present invention and the medium of the present invention.

According to another aspect of the present invention, there is further provided a method for promoting the growth of a lactic acid coccus, comprising the step of adding a dicarboxylic acid and/or a salt thereof to a medium for a lactic acid coccus. The growth promoting method of the present invention can be carried out according to the descriptions regarding the growth promoter of the present invention.

According to another aspect of the present invention, there is provided use of a dicarboxylic acid and/or a salt thereof as a growth promoter for a lactic acid coccus, for promoting the growth of a lactic acid coccus, or in the method for promoting the growth of a lactic acid coccus of the present invention. The use of the present invention can be carried out according to the descriptions regarding the growth promoter and growth promoting method of the present invention.

### EXAMPLES

Hereinafter, the present invention will be described in more detail by way of the following examples, but is not limited thereto.

### Example 1: Evaluation (1) of growth promoting effect of dicarboxylic acid on lactic acid coccus

In Example 1, the growth promoting effect of dicarboxylic acids on a lactic acid coccus was tested.

### (1) Method

### A. Bacterial cell

*A Lactococcus lactis* subsp. *lactis* JCM5805 strain was used as the lactic acid coccus.

### B. Cultivation method

### (i) Anaerobic cultivation

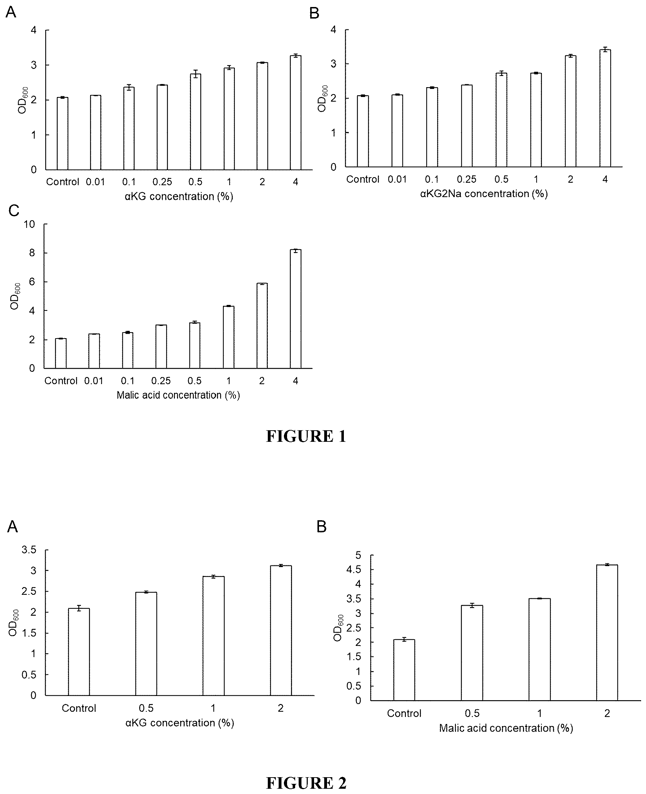

The medium used was MRS medium (MRS BROTH, CODE: CM0359, Oxoid; the composition thereof is indicated in Table 1; the same applies in Examples 3 to 6 of the present specification). As the dicarboxylic acids, α-ketoglutaric acid (FUJIFILM Wako Pure Chemical Corporation), disodium α-ketoglutarate (sometimes referred to as "αKG2Na" herein) (FUJIFILM Wako Pure Chemical Corporation) and malic acid (Tokyo Chemical Industry Co., Ltd.) were each added to the medium at a concentration (0.01 to 4.0% by mass) as shown in FIG. 1, and sodium hydroxide was added so as to attain the same pH as that of a control (dicarboxylic acid-free medium) (pH: 6.2 ± 0.2). Next, the bacterial cells were inoculated (seeded) in a 15-ml conical tube (FALCON) so that the concentration of the bacterial cells was 0.1% (v/v) with respect to 10 ml of the prepared medium, and cultivated at 30°C for 24 hours in an incubator (TOKYO RIKAKIKAI CO., LTD.) (static cultivation).

### [Table 1]

**Table 1: Composition of MRS medium**

| | g/L |
|---|---|
| Peptone | 10.0 |
| Lab-Lemco powder (beef extract) | 8.0 |
| Yeast extract | 4.0 |
| Glucose | 20.0 |
| Sorbitan oleate | 1 ml |
| Dipotassium hydrogen phosphate | 2.0 |
| Sodium acetate trihydrate | 5.0 |
| Triammonium citrate | 2.0 |
| Magnesium sulfate heptahydrate | 0.2 |
| Manganese sulfate tetrahydrate | 0.05 |
| Purified water | Balance |

### (ii) Aerobic cultivation

The medium used was MRS medium. As the dicarboxylic acids, α-ketoglutaric acid, disodium α-ketoglutarate, and malic acid were each added to the medium at a concentration 0.5 to 2.0% by mass) as shown in FIG. 2, and sodium hydroxide was added so as to attain the same pH as that of a control (dicarboxylic acid-free medium) (pH: 6.2 ± 0.2). Next, the bacterial cells were inoculated (seeded) so that the concentration of the bacterial cells was 0.1% (v/v) with respect to 10 ml of the medium, and the medium inoculated with the bacterial cells was placed in an automatic cultivation searching device OT-201 (Oriental Instruments; sometimes referred to as "bioplotter" herein), and cultivated at 30°C for 24 hours (stirring intensity condition: Medium).

### C. Measurement of bacterial count

The bacteria were counted by measuring the OD600 (turbidity) of the medium using an absorbance meter (Biochrom GeneQuant 1300).

### D. Statistical analysis

Measured values were expressed as mean ± standard error. One-way analysis of variance was followed by Tukey's test, and cases where p<0.05 relative to the control group were evaluated as having a significant difference.

### (2) Result

The results were as shown in FIGS. 1 and 2. It was confirmed that, in the anaerobic cultivation, the growth of the lactic acid coccus was significantly promoted in a concentration-dependent manner in the media added with αKG (FIG. 1A) and malic acid (FIG. 1C) as compared with the control (dicarboxylic acid-free medium). In addition, it was confirmed that the growth of the lactic acid coccus was significantly promoted in a concentration-dependent manner at a concentration of 0.1 to 4% by mass in the medium added with αKG2Na (FIG. 1B) as compared with the control (dicarboxylic acid-free medium). Also at 0.01% by mass, the growth of the lactic acid coccus tended to be promoted. It was confirmed that, similarly in the aerobic cultivation, the growth of the lactic acid coccus was significantly promoted in a concentration-dependent manner in the media added with αKG (FIG. 2A) and malic acid (FIG. 2B) as compared with the control (dicarboxylic acid-free medium). It was confirmed that the addition of either of the dicarboxylic acid and salt thereof to the medium free of meat extract similarly promoted the growth of the lactic acid coccus.

### Example 2: Evaluation (2) of growth promoting effect of dicarboxylic acid on lactic acid coccus

In Example 2, the growth promoting effect of dicarboxylic acids on a lactic acid coccus was tested using a medium different from the medium used in Example 1.

### (1) Method

### A. Bacterial cell

The lactic acid coccus described in Example 1, item (1), A was used.

### B. Cultivation method

### (i) Anaerobic cultivation

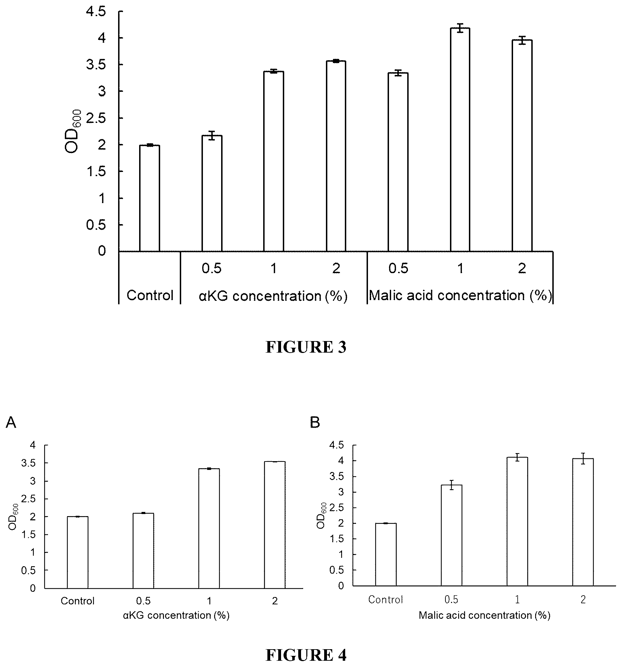

The medium used was a medium obtained by adding 20 g/L of glucose to M17 medium (Difco M17 Broth, BD; the composition thereof is indicated in Table 2; the same applies hereinafter in the present specification) (sometimes referred to as "GM17 medium" herein). As the dicarboxylic acids, α-ketoglutaric acid and malic acid were each added to the medium at a concentration (0.5 to 2% by mass) as shown in FIG. 3, and sodium hydroxide was added so as to attain the same pH as that of a control (dicarboxylic acid-free medium) (pH: 6.5 ± 0.2). Next, the bacterial cells were inoculated (seeded) in a 15-ml conical tube (FALCON) so that the concentration of the bacterial cells was 0.1% (v/v) with respect to 10 ml of the prepared medium, and cultivated at 30°C for 24 hours in an incubator (TOKYO RIKAKIKAI CO., LTD.) (static cultivation).

### [Table 2]

**Table 2: Composition of M17 medium**

| | Per 950 mL of purified water |
|---|---|
| Pancreatic digest of casein | 5.0 g |
| Soy peptone | 5.0 g |
| Beef extract | 5.0 g |
| Yeast extract | 2.5 g |
| Ascorbic acid | 0.5 g |
| Magnesium sulfate | 0.25 g |
| Disodium β-glycerophosphate | 19.0 g |

### (ii) Aerobic cultivation

The medium used was GM17 medium. As the dicarboxylic acids, α-ketoglutaric acid and malic acid were each added to the medium at a concentration (0.5 to 2% by mass) as shown in FIG. 4, and sodium hydroxide was added so as to attain the same pH as that of a control (dicarboxylic acid-free medium) (pH: 6.5 ± 0.2). Next, the bacterial cells were inoculated (seeded) so that the concentration of the bacterial cells was 0.1% (v/v) with respect to 10 ml of the prepared medium, and the medium inoculated with the bacterial cells was placed in an automatic cultivation searching device OT-201 (Oriental Instruments; sometimes referred to as "bioplotter" herein), and cultivated at 30°C for 24 hours (stirring intensity condition: Medium).

### C. Measurement of bacterial count

Measurement was performed in a similar manner as described in Example 1, item (1), C.

### D. Statistical analysis

Analysis was performed in a similar manner as described in Example 1, item (1), D.

### (2) Result

The results were as shown in FIGS. 3 and 4. It was confirmed that, in the anaerobic cultivation, the growth of the lactic acid coccus was significantly promoted in the media added with αKG (FIG. 3A) and malic acid (FIG. 3B) as compared with the control (dicarboxylic acid-free medium). It was confirmed that, similarly in the aerobic cultivation, the growth of the lactic acid coccus was promoted in a concentration-dependent manner in the media added with αKG (FIG. 4A) and malic acid (FIG. 4B) as compared with the control (dicarboxylic acid-free medium). From the results of Examples 1 and 2, it was confirmed that the dicarboxylic acid significantly promoted the growth of the lactic acid coccus regardless of the type of medium.

### Example 3: Evaluation (3) of growth promoting effect of dicarboxylic acid on lactic acid coccus

In Example 3, the growth promoting effect of α-ketoglutaric acid on various lactic acid cocci was tested.

### (1) Method

### A. Bacterial cell

The lactic acid cocci indicated in Table 3 were used as the bacterial cells.

### [Table 3]

**Table 3: List of lactic acid cocci**

| Name of bacterial strain | Bacterial species |
|---|---|
| NBRC100934 | *Lactococcus garvieae* |
| JCM16167 | *Lactococcus lactis* subsp. *cremoris* |
| NBRC100676 | *Lactococcus lactis* subsp. *cremoris* |
| JCM1180 | *Lactococcus lactis* subsp. *hordniae* |
| JCM11040 | *Lactococcus lactis* subsp. *hordniae* |
| NBRC12007 | *Lactococcus lactis* subsp. *lactis* |
| NRIC1150 | *Lactococcus lactis* subsp. *lactis* |
| JCM20101 | *Lactococcus lactis* subsp. *lactis* |
| NBRC12455 | *Leuconostoc lactis* |
| JCM7638 | *Lactococcus lactis* subsp. *lactis* |
| ATCC11454 | *Lactococcus lactis* subsp. *lactis* |

### B. Cultivation method

Cultivation was performed in a similar manner as described in Example 1, item (1), B (i), except that α-ketoglutaric acid was added, as the dicarboxylic acid, to the medium at a concentration of 2.0% by mass (anaerobic cultivation).

### C. Measurement of bacterial count

Measurement was performed in a similar manner as described in Example 1, item (1), C.

### D. Statistical analysis

Measured values were expressed as mean ± standard error. A Student-t test was performed, and cases where p<0.05 relative to the control group were evaluated as having a significant difference.

### (2) Result

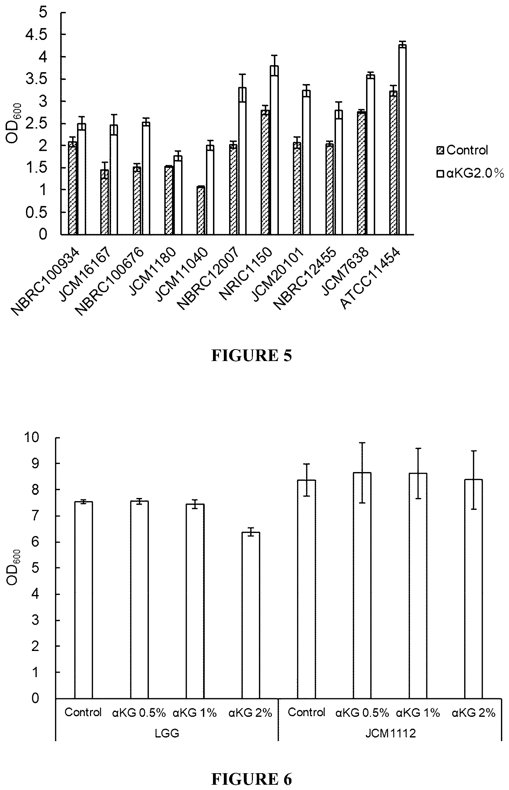

The results were as shown in FIG. 5. The growth of the lactic acid cocci of *Lactococcus garvieae, Lactococcus lactis* subsp. *cremoris, Lactococcus lactis* subsp. *hordniae, Lactococcus lactis* subsp. *lactis* and *Leuconostoc lactis* was confirmed to be significantly promoted by the dicarboxylic acid as compared with the control (dicarboxylic acid-free medium), regardless of the bacterial strain.

### Example 4: Evaluation of growth promoting effect of dicarboxylic acid on lactic acid-producing bacillus

In Example 4, the growth promoting effect of α-ketoglutaric acid on lactic acid-producing bacilli belonging to the genus *Lactobacillus* was tested.

### (1) Method

### A. Bacterial cell

The lactic acid-producing bacilli of the genus *Lactobacillus* indicated in Table 4 were used as the bacterial cells.

### [Table 4]

**Table 4: List of lactic acid-producing bacilli**

| Name of bacterial strain | Bacterial species |
|---|---|
| LGG | *Lactobacillus rhamnosus* GG |
| JCM1112 | *Lactobacillus reuteri* |

### B. Cultivation method

Cultivation was performed in a similar manner as described in Example 1, item (1), B (i), except that α-ketoglutaric acid was added, as the dicarboxylic acid, to the medium at a concentration (0.5 to 2% by mass) as shown in FIG. 6 (anaerobic cultivation).

### C. Measurement of bacterial count

Measurement was performed in a similar manner as described in Example 1, item (1), C.

### D. Statistical analysis

Analysis was performed in a similar manner as described in Example 1, item (1), D.

### (2) Result

The results were as shown in FIG. 6. The promotion of the growth of the lactic acid-producing bacillus belonging to the genus *Lactobacillus* by the dicarboxylic acid was not observed, as compared with the control (dicarboxylic acid-free medium).

### Example 5: Evaluation of growth promoting effect of tricarboxylic acid on lactic acid coccus

In Example 5, the growth promoting effect of a tricarboxylic acid on a lactic acid coccus was tested.

### (1) Method

### A. Bacterial cell

A similar lactic acid coccus to that described in Example 1, item (1), A was used.

### B. Cultivation method

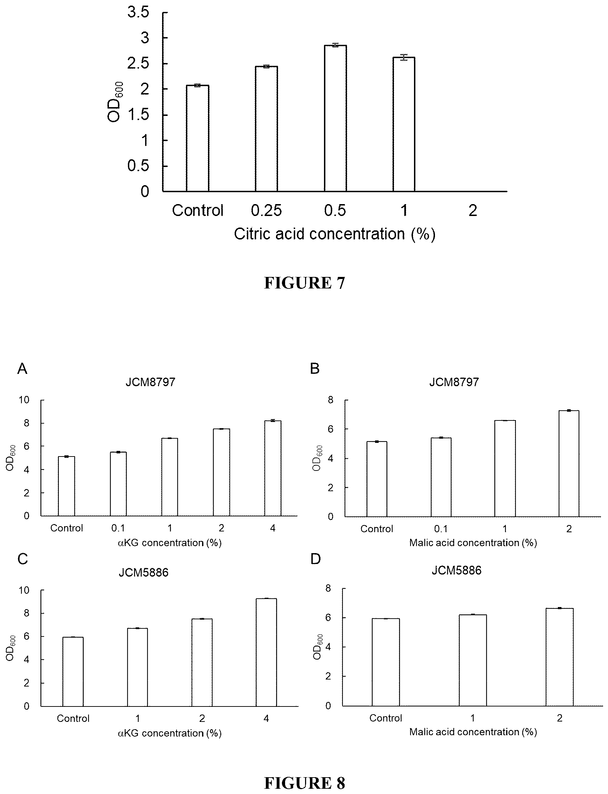

Cultivation was performed in a similar manner as described in Example 1, item (1), B (i), except that citric acid (FUJIFILM Wako Pure Chemical Corporation) was added as a tricarboxylic acid, in place of the dicarboxylic acid, to the medium at a concentration (0.25 to 1% by mass) as shown in FIG. 7 (anaerobic cultivation).

### C. Measurement of bacterial count

Measurement was performed in a similar manner as described in Example 1, item (1), C.

### D. Statistical analysis

Analysis was performed in a similar manner as described in Example 1, item (1), D.

### (2) Result

The results were as shown in FIG. 7. It was confirmed that the growth of the lactic acid coccus was significantly promoted by citric acid as compared with the control (dicarboxylic acid-free medium), but the dicarboxylic acid had a greater degree of growth promoting effect than that of the citric acid.

### Example 6: Evaluation (4) of growth promoting effect of dicarboxylic acid on lactic acid coccus

In Example 6, the growth promoting effect of dicarboxylic acids on lactic acid cocci belonging to the genus *Pediococcus* was tested.

### (1) Method

### A. Bacterial cell

The lactic acid cocci of the genus *Pediococcus* indicated in Table 5 were used as the bacterial cells.

### [Table 5]

**Table 5: List of lactic acid cocci**

| Name of bacterial strain | Bacterial species |
|---|---|
| JCM8797 | *Pediococcus acidilactici* |
| JCM5886 | *Pediococcus damnosus* |

### B. Cultivation method

Cultivation was performed in a similar manner as described in Example 1, item (1), B (i), except that α-ketoglutaric acid (concentrations shown in FIG. 8A (0.1 to 4% by mass) and FIG. 8C (1 to 4% by mass)) and malic acid (concentrations shown in FIG. 8B (0.1 to 2% by mass) and FIG. 8D (1 to 2% by mass)) were each added, as the dicarboxylic acids, to the medium (anaerobic cultivation).

### C. Measurement of bacterial count

Measurement was performed in a similar manner as described in Example 1, item (1), C.

### D. Statistical analysis

Analysis was performed in a similar manner as described in Example 1, item (1), D.

### (2) Result

The results were as shown in FIG. 8. It was confirmed that the growth of the lactic acid cocci belonging to the genus *Pediococcus* was significantly promoted in a concentration-dependent manner in the media added with αKG (FIGS. 8A and 8C) and malic acid FIGS. 8B and 8D as compared with the control (dicarboxylic acid-free medium).

### Example 7: Evaluation (5) of growth promoting effect of dicarboxylic acid on lactic acid coccus

In Example 7, the growth promoting effect of a dicarboxylic acid on lactic acid cocci in a meat extract-free medium was tested.

### (1) Method

### A. Bacterial cell

The lactic acid cocci indicated in Table 6 were used as the bacterial cells.

### [Table 6]

**Table 6: List of lactic acid cocci**

| Name of bacterial strain | Bacterial species |
|---|---|
| JCM5805 | *Lactococcus lactis* subsp. *lactis* |
| NRBC100934 | *Lactococcus garvieae* |
| JCM16167 | *Lactococcus lactis* subsp. *cremoris* |
| NBRC100676 | *Lactococcus lactis* subsp. *cremoris* |
| JCM1180 | *Lactococcus lactis* subsp. *hordniae* |
| JCM11040 | *Lactococcus lactis* subsp. *hordniae* |
| NBRC12007 | *Lactococcus lactis* subsp. *lactis* |
| NRIC1150 | *Lactococcus lactis* subsp. *lactis* |
| JCM20101 | *Lactococcus lactis* subsp. *lactis* |
| NBRC12455 | *Leuconostoc lactis* |
| JCM7638 | *Lactococcus lactis* subsp. *lactis* |
| ATCC11454 | *Lactococcus lactis* subsp. *lactis* |

### B. Cultivation method

The medium used was MRS medium adjusted so as to have a composition as indicated in Table 7 (adjusted to have a pH of 6.2 by adding a phosphoric acid). As the dicarboxylic acid, α-ketoglutaric acid was added to the medium at a concentration of 2.0% by mass, and sodium hydroxide was added so as to attain the same pH as that of a control (dicarboxylic acid-free medium) (pH: 6.2 ± 0.2). Next, the bacterial cells were inoculated (seeded) in a 15-ml conical tube (FALCON) so that the concentration of the bacterial cells was 0.1% (v/v) with respect to 10 ml of the prepared medium, and cultivated at 30°C for 24 hours in an incubator (TOKYO RIKAKIKAI CO., LTD.) (static cultivation).

### [Table 7]

**Table 7: Composition of MRS medium (free of meat extract)**

| | g/L |
|---|---|
| Peptone (BD) | 10.0 |
| Yeast extract (BD) | 4.0 |
| Glucose (FUJIFILM Wako Pure Chemical Corporation) | 20.0 |
| Sorbitan oleate (Kanto Chemical Industry Co., Ltd.) | 1 ml |
| Dipotassium hydrogen phosphate (FUJIFILM Wako Pure Chemical Corporation) | 2.0 |
| Sodium acetate trihydrate (FUJIFILM Wako Pure Chemical Corporation) | 5.0 |
| Triammonium citrate (Kanto Chemical Industry Co., Ltd.) | 2.0 |
| Magnesium sulfate heptahydrate (FUJIFILM Wako Pure Chemical Corporation) | 0.2 |
| Manganese sulfate pentahydrate (Kishida Chemical Co., | 0.05 |
| Ltd.) | |
| Purified water | Balance |

### C. Measurement of bacterial count

Measurement was performed in a similar manner as described in Example 1, item (1), C.

### D. Statistical analysis

Analysis was performed in a similar manner as described in Example 1, item (1), D.

### (2) Result

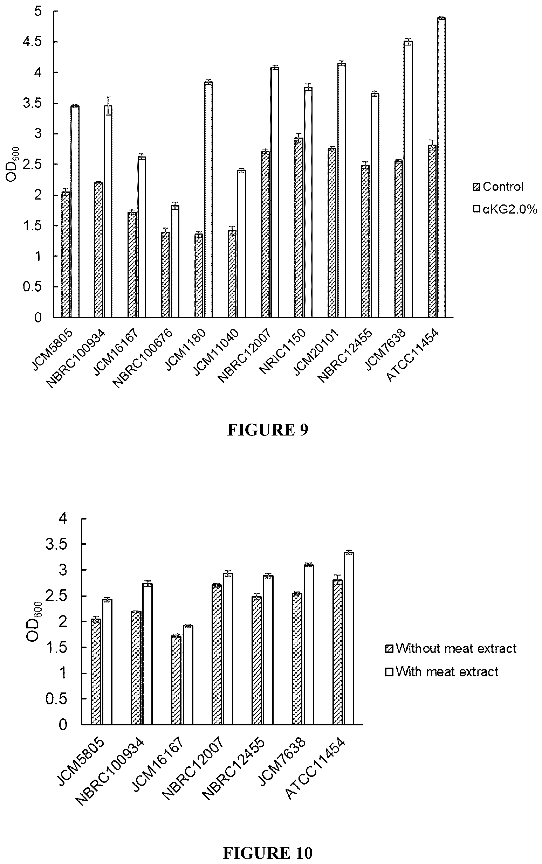

The results were as shown in FIG. 9. The growth of the lactic acid cocci of *Lactococcus lactis* subsp. *lactis, Lactococcus garvieae, Lactococcus lactis* subsp. *cremoris, Lactococcus lactis* subsp. *hordniae* and *Leuconostoc lactis* was confirmed to be significantly promoted also in the meat extract-free medium by addition of the dicarboxylic acid as compared with the control (dicarboxylic acid-free medium).

### Example 8: Evaluation of influence of meat extract in medium on growth of lactic acid coccus

In Example 8, the influence of meat extract in a medium on the growth of lactic acid cocci was tested.

### (1) Method

### A. Bacterial cell

The lactic acid cocci indicated in Table 8 were used as the bacterial cells.

### [Table 8]

**Table 8: List of lactic acid cocci**

| Name of bacterial strain | Bacterial species |
|---|---|
| JCM5805 | *Lactococcus lactis* subsp. *lactis* |
| NRBC 100934 | *Lactococcus garvieae* |
| JCM16167 | *Lactococcus lactis* subsp. *cremoris* |
| NBRC 12007 | *Lactococcus lactis* subsp. *lactis* |
| NBRC12455 | *Leuconostoc lactis* |
| JCM7638 | *Lactococcus lactis* subsp. *lactis* |
| ATCC11454 | *Lactococcus lactis* subsp. *lactis* |

### B. Cultivation method

The meat extract-free medium used was MRS medium adjusted so as to have a composition as indicated in Table 7 (adjusted to have a pH of 6.2 by adding a phosphoric acid because the initial pH was around 7.0). The meat extract-containing medium used was MRS medium adjusted so as to have a composition as indicated in Table 9 (adjusted to have a pH of 6.2 by adding a phosphoric acid). Next, the bacterial cells were inoculated (seeded) in a 15-ml conical tube (FALCON) so that the concentration of the bacterial cells was 0.1% (v/v) with respect to 10 ml of the prepared medium, and cultivated at 30°C for 24 hours in an incubator (TOKYO RIKAKIKAI CO., LTD.) (static cultivation).

### [Table 9]

**Table 9: Composition of MRS medium (containing meat extract)**

| | g/L |
|---|---|
| Peptone (BD) | 10.0 |
| Lab-Lemco powder (beef extract) (Oxoid) | 8.0 |
| Yeast extract (BD) | 4.0 |
| Glucose (FUJIFILM Wako Pure Chemical Corporation) | 20.0 |
| Sorbitan oleate (Kanto Chemical Industry Co., Ltd.) | 1 ml |
| Dipotassium hydrogen phosphate (FUJIFILM Wako Pure Chemical Corporation) | 2.0 |
| Sodium acetate trihydrate (FUJIFILM Wako Pure Chemical Corporation) | 5.0 |
| Triammonium citrate (Kanto Chemical Industry Co., Ltd.) | 2.0 |
| Magnesium sulfate heptahydrate (FUJIFILM Wako Pure Chemical Corporation) | 0.2 |
| Manganese sulfate pentahydrate (Kishida Chemical Co., Ltd.) | 0.05 |
| Purified water | Balance |

### C. Measurement of bacterial count

Measurement was performed in a similar manner as described in Example 1, item (1), C.

### D. Statistical analysis

Analysis was performed in a similar manner as described in Example 1, item (1), D.

### (2) Result

The results were as shown in FIG. 10. The test on the influence on the growth of the lactic acid cocci was tested using the media different only in the presence or absence of meat extract verified that the growth of the lactic acid cocci was significantly higher in the meat extract-containing medium than in the meat extract-free medium. In addition, from the results of Examples 7 and 8, it was confirmed that the medium obtained by adding the dicarboxylic acid to the meat extract-free medium has a higher OD₆₀₀ value than the meat extract-containing medium (without addition of dicarboxylic acid), resulting in a higher growth promoting effect of the dicarboxylic acid on the lactic acid cocci (FIGS. 9 and 10).

## Claims

1. A growth promoter for a lactic acid coccus, comprising a dicarboxylic acid and/or a salt thereof as an active ingredient.

2. The growth promoter according to claim 1, wherein the dicarboxylic acid is an aliphatic dicarboxylic acid having 4 to 6 carbon atoms.

3. The growth promoter according to claim 1 or 2, wherein the dicarboxylic acid and/or salt thereof is one or more selected from the group consisting of malic acid, α-ketoglutaric acid and salts thereof.

4. The growth promoter according to any one of claims 1 to 3, wherein the lactic acid coccus is a bacterium belonging to the genus *Lactococcus.*

5. The growth promoter according to any one of claims 1 to 4, wherein the lactic acid coccus is *Lactococcus lactis* subsp. *lactis* JCM5805.

6. The growth promoter according to any one of claims 1 to 5, wherein the dicarboxylic acid and/or salt thereof is used at a concentration of 0.01 to 8% by mass in a medium.

7. The growth promoter according to any one of claims 1 to 6, which is added to a medium that is substantially free of meat extract.

8. A medium for a lactic acid coccus, comprising a dicarboxylic acid and/or a salt thereof.

9. The medium according to claim 8, which contains the dicarboxylic acid and/or salt thereof at a concentration of 0.01 to 8% by mass.

10. The medium according to claim 8 or 9, which is substantially free of meat extract.

11. A method for cultivation of a lactic acid coccus, comprising the step of cultivation of a lactic acid coccus in the medium according to any one of claims 8 to 10.

12. A method for promoting the growth of a lactic acid coccus, comprising the step of adding a dicarboxylic acid and/or a salt thereof to a medium for a lactic acid coccus.

13. Use of a dicarboxylic acid and/or a salt thereof as a growth promoter for a lactic acid coccus.
